(19)

Europäisches
Patentamt

European
Patent Office

Office européen
des brevets

(11) **EP 2 131 197 B1**

(12) **EUROPÄISCHE PATENTSCHRIFT**

(45) Veröffentlichungstag und Bekanntmachung des Hinweises auf die Patenterteilung:
**15.09.2010 Patentblatt 2010/37**

(51) Int Cl.:
*G01N 33/557* (2006.01)      *G01N 21/64* (2006.01)

(21) Anmeldenummer: **08010289.0**

(22) Anmeldetag: **05.06.2008**

(54) **Verfahren zum Bestimmen eines Analyten in einer Flüssigkeitsprobe und Analysevorrichtung**

Device for determining an analyte in a fluid sample and analysis device

Procédé de détermination d'une analyse dans un échantillon de liquide et dispositif d'analyse

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**09.12.2009 Patentblatt 2009/50**

(73) Patentinhaber:
• **F.HOFFMANN-LA ROCHE AG**
  **4070 Basel (CH)**
• **Roche Diagnostics GmbH**
  **68305 Mannheim (DE)**
  Benannte Vertragsstaaten:
  **DE**

(72) Erfinder:
• **Händler, Dr. Erich**
  **68623 Lampertheim (DE)**
• **Oranth, Dr. Norbert**
  **69493 Hirschberg (DE)**

(74) Vertreter: **Bittner, Thomas L.**
  **Forrester & Boehmert**
  **Pettenkoferstrasse 20-22**
  **80336 München (DE)**

(56) Entgegenhaltungen:
**EP-A- 0 690 306**

• **HOLBROOK J J: "Protein fluorescence of lactate hydrogenase" BIOCHEMICAL JOURNAL, Bd. 128, Nr. 4, 1972, Seiten 921-931, XP002503387 ISSN: 0264-6021**
• **SCHULT K ET AL: "Disposable optical sensor chip for medical diagnostics: new ways in bioanalysis" ANALYTICAL CHEMISTRY, Bd. 71, Nr. 23, 1. Dezember 1999 (1999-12-01), Seiten 5430-5435, XP000902374 ISSN: 0003-2700**
• **HANSEN E H: "Principles and applications of flow injection analysis in biosensors" JOURNAL OF MOLECULAR RECOGNITION, Bd. 9, Nr. 5-6, 1996, Seiten 316-325, XP002503388 ISSN: 0952-3499**

**Beschreibung**

Die Erfindung betrifft ein Verfahren zum Bestimmen eines Analyten in einer Flüssigkeitsprobe, insbesondere einer Körperflüssigkeitsprobe, sowie eine Analysevorrichtung und ein Computerprogramm-Produkt.

Hintergrund der Erfindung

Derartige Verfahren und Vorrichtungen dienen dem analytischen Nachweis eines oder mehrerer Analyte in einer Flüssigkeitsprobe. Ergänzend oder alternativ kann das Bestimmen eines Analyten in der Flüssigkeitsprobe neben dem grundsätzlichen Nachweis des Analyten an sich auch das Messen analytspezifischer Eigenschaften in der Flüssigkeitsprobe umfassen. Hierbei wird üblicherweise die zu untersuchende Flüssigkeitsprobe auf ein Testsystem oder -element mit mehreren Analyt-Nachweiszonen gegeben, die auch als Mess- oder Untersuchungsbereiche bezeichnet werden. Beispielsweise bewegt sich die aufgegebene Flüssigkeitsprobe hierbei entlang einer geraden Linie, auf der die mehreren Messbereiche oder Nachweiszonen angeordnet sind. In den Messbereichen, die eine Kreis-, eine Rechteck- oder eine Strichform aufweisen können, sind analytspezifische Fängerstrukturen angeordnet, so dass in den Messbereichen jeweils ein Teil des Analyten aus der Flüssigkeitsprobe gebunden wird.

Üblicherweise sind der oder die Analyte direkt oder über Antikörper gelabelt und hierdurch für eine anschließende optische Auswertung des Testsystems vorbereitet. Gegebenenfalls kann eine Aufbereitung des Testsystems oder -elementes auch das Spülen oder Waschen mit einer Puffer-, einer Verdünnungs- oder einer Waschlösung umfassen. Schließlich kann das Testsystem, welches beispielsweise ein trockenchemisches Testelement ist, dann mittels optischen Abtasten ausgewertet werden, um insbesondere das Auftreten eines oder mehrerer bestimmter Analyte in den Messbereichen festzustellen.

Beim optischen Abtasten, was auch als optisches Scannen bezeichnet wird, werden Messlichtstrahlen, welche in den abgetasteten Untersuchungsbereichen entstehen, nach dem Verlassen der Untersuchungsbereiche mit einer Detektoreinrichtung erfasst. Die Messlichtstrahlen können erzeugt werden, indem Testlichtstrahlen, die ihrerseits von einer geeigneten monochromatischen oder polychromatischen Lichtquelle erzeugt werden, auf die mit zu messenden, optisch aktiven Substanzen beladenen Messbereiche eingestrahlt werden. In den Messbereichen wechselwirkt dann das Licht der Testlichtstrahlen mit den optisch aktiven Substanzen, so dass sich eine entsprechende Änderung der optischen Eigenschaften der Testlichtstrahlen ergibt, welche den Messbereich dann als Messlichtstrahlen verlassen. Beim Einstrahlen von Testlicht können als optische Eigenschaften der Messlichtstrahlen die Absorption, die Transmission, die Reflexion oder auch die Fluoreszenz untersucht werden. Messlichtstrahlen können darüber hinaus auf einer Lumineszenz der optisch aktiven Substanzen in den Messbereichen beruhen.

Beim optischen Abtasten des Testelementes / -systems können beispielsweise das Testelement und die Detektoreinrichtung, mit welcher die Messlichtstrahlen erfasst werden, oder das Testelement und die Lichtquelle relativ zueinander verlagert, um so Messbereich nach Messbereich optisch zu analysieren.

Mithilfe der optischen Messverfahren wie Absorption, Fluoreszenz, Transmission oder Reflexion werden die Messbereiche optisch untersucht, um die Bindung des Analyten in der jeweiligen Zone zu analysieren. Hierbei werden die so genannten Testlichtstrahlen auf den Messbereich gegeben, die dann im Messbereich mit optischen Labeln wechselwirken, die an die analytspezifischen Fängerstrukturen binden, wodurch die Messlichtstrahlen entstehen, die mit Hilfe einer optischen Detektoreinrichtung erfasst werden. Als solche optischen Detektoreinrichtungen dienen beispielsweise eine Fotodiode oder eine Fotodiodenanordnung. Die für die Messlichtstrahlen gemessenen Signale werden herangezogen, um beispielsweise eine Konzentration oder andere Eigenschaften des Analyten in der Flüssigkeitsprobe zu ermitteln.

Die erfassten Messlichtstrahlen hängen üblicherweise von der Bindungseffizienz ab, mit der ein an dem Messbereich oder der Nachweiszone vorbei fließender Komplex des Analyten dort an die Fängerstrukturen bindet. Die Bindungseffzienz ihrerseits ist im Allgemeinen abhängig von verschiedenen Eigenschaften wie der Art des für den Messbereich genutzten Substrats (Membran), der individuellen Flüssigkeitsprobe, den analytspezifischen Fängerstrukturen oder der Temperatur. Die Bindungseffizienz ist deshalb sehr unterschiedlich für verschiedene Mess- oder Analysesituationen. Dieses bedeutet, dass sich üblicherweise eine Variation der Bindungseffizienz direkt auf das Analyseergebnis auswirkt.

Das Dokument EP 0 690 306 A1 beschreibt ein Verfahren und eine Vorrichtung zur Untersuchung von spezifischen Bindungen. Ein Signalsubstanzerzeuger erzeugt basierend auf einer spezifischen Bindungsreaktion eine Signalsubstanz, welche zusammen mit einer flüssigen Probe in einer bestimmten Richtung durch einen Kanal fließt. Die Signalsubstanz wird mit mehreren Nachweismitteln an verschiedenen Positionen in der Fließrichtung untersucht.

Das Dokument J. J. Holbrook "Protein Fluorescence of Lactate Dehydrogenase" (Biochem. J. Bd. 128, S. 921 (1972)) beschreibt einen nichtlinearen Abfall der Proteinfluoreszenz von Laktatdehydrogenase mit einem Anstieg des Anteils von Koenzymbindungsplätzen, die mit NADH (Nicotinamidadenindinukleotid) besetzt sind. Die relative Proteinfluoreszenz von Molekülen mit $j$ Liganden bildet eine geometrische Reihe.

Zusammenfassung der Erfindung

Aufgabe der Erfindung ist es, ein Verfahren zum Bestimmen eines Analyten in einer Flüssigkeitsprobe sowie eine Analysevorrichtung vorzuschlagen, mit denen eine verbesserte Analysemöglichkeit für den Analyten erreicht ist.

Diese Aufgabe wird erfindungsgemäß durch ein Verfahren zum Bestimmen eines Analyten in einer Flüssigkeitsprobe, insbesondere einer Körperflüssigkeitsprobe, nach dem unabhängigen Anspruch 1 sowie eine Analysevorrichtung nach dem unabhängigen Anspruch 4 gelöst. Weiterhin ist ein Computerprogramm-Produkt zum Bestimmen eines Analyten in einer Flüssigkeitsprobe in einer Auswerteeinrichtung nach dem unabhängigen Anspruch 10 geschaffen. Vorteilhafte Ausgestaltungen der Erfindung sind Gegenstand von abhängigen Unteransprüchen.

Die Erfindung umfasst den Gedanken eines Verfahrens zum Bestimmen eines Analyten in einer Flüssigkeitsprobe, insbesondere einer Körperflüssigkeitsprobe, mit Hilfe einer Analysevorrichtung, bei dem von einer von der Analyseeinrichtung umfassten Auswerteeinrichtung, ein erstes Messsignal $s_i$ ($i$ = 0, 1, ...) und ein zweites Messsignal $s_j$ ($j$ = 1, 2, ...; $j > i$) für ein Testsystem mit mehreren nacheinander angeordneten und von einer Flüssigkeitsprobe mit einem Analyten nach einem Aufgeben auf das Testsystem durchlaufbaren Messbereich empfangen werden, wobei das erste Messsignal $s_i$ einen in einem ersten Messbereich gebundenen Teil des Analyten und das zweite Messsignal $s_j$ einen in einem zweiten Messbereich, der dem ersten Messbereich auf dem Testsystem nachgelagert ist, gebundenen Teil des Analyten nach der Aufgabe der Flüssigkeitsprobe auf das Testsystem anzeigen, von der Auswerteeinrichtung ein mit der Konzentration des Analyten in der Flüssigkeitsprobe korrelierendes Konzentrationsmaß $N$ gemäß der folgenden Messsignalverknüpfung ermittelt wird:

$$N = \frac{(s_j)^{j+2}}{\left[s_i - \left((s_i)^{j-i-1} \cdot s_j\right)^{\frac{1}{j-i}}\right] \cdot \left((s_i)^{j-i-1} \cdot s_j\right)^{\frac{i}{j-1}}},$$

und

von der Auswerteeinrichtung das Konzentrationsmaß $N$ nach einer wahlweisen Weiterverarbeitung ausgegeben wird.

Nach einem weiteren Aspekt der Erfindung ist eine Analysevorrichtung zum Bestimmen eines Analyten in einer Flüssigkeitsprobe, insbesondere einer Körperflüssigkeitsprobe, mit einer Auswerteeinrichtung geschaffen, die konfiguriert ist, ein erstes Messsignal $s_i$ ($i$ = 0, 1, ...) und ein zweites Messsignal $s_j$ ($j$ = 1, 2, ...; $j > i$) für ein Testsystem mit mehreren nacheinander angeordneten und von einer Flüssigkeitsprobe mit einem Analyten nach einem Aufgeben auf das Testsystem durchlaufbaren Messbereichen zu empfangen, wobei das erste Messsignal $s_i$ einen in einem ersten Messbereich gebundenen Teil des Analyten und das zweite Messsignal $s_j$ einen in einem zweiten Messbereich, der dem ersten Messbereich auf dem Testsystem nachgelagert ist, gebundenen Teil des Analyten nach der Aufgabe der Flüssigkeitsprobe auf das Testsystem anzeigen und wobei die Auswerteeinrichtung weiterhin konfiguriert ist, ein mit der Konzentration des Analyten in der Flüssigkeitsprobe korrelierendes Konzentrationsmaß $N$ gemäß der folgenden Messsignalverknüpfung zu ermitteln und auszugeben:

$$N = \frac{(s_j)^{j+2}}{\left[s_i - \left((s_i)^{j-i-1} \cdot s_j\right)^{\frac{1}{j-i}}\right] \cdot \left((s_i)^{j-i-1} \cdot s_j\right)^{\frac{i}{j-1}}}.$$

Ein wesentlicher Vorteil, welcher mit der Erfindung gegenüber dem Stand der Technik erreicht ist, besteht darin, dass die Ermittlung des Konzentrationsmaßes durch die Auswerteeinrichtung unabhängig von der Bindungseffizienz für den Analyten der Flüssigkeitsprobe in den Messbereichen des Testsystems ist. Die vorgeschlagene Signalverarbeitung eliminiert sozusagen den Bindungseffizienzeinfluss. Auf diese Weise hängt die Bestimmung des Konzentrationsmaßes für den Analyten in der Flüssigkeitsprobe weit weniger von den sich von Situation zu Situation häufig ändernden Analysebedingungen ab.

Im hier verstandenen Sinne korreliert das Konzentrationsmaß $N$ mit der Konzentration derart, dass eine Zunahme der Konzentration auch eine Zunahme des Konzentrationsmaßes bewirkt. In gleicher Weise verhält es sich mit einer Abnahme des Konzentrationsmaßes bei Abnahme der Konzentration. Der Korrelation kann linearer oder nichtlinearer Natur sein. Hierbei muss ein funktioneller Zusammenhang zwischen Konzentration und Konzentrationsmaß nicht in mathematisch exakter Form bekannt sein; vielmehr kann die tatsächlich Konzentration auch unter Verwendung einer experimentell ermittelten Eich- oder Kalibrierungskurve ermittelt werden.

Die vorgeschlagene Technologie zur Analytbestimmung zeichnet sich weiterhin dadurch aus, dass zur Auswertung der Testsystem basierten Analyse Messsignale für beliebige Messbereiche des Testsystems herangezogen werden können. Die Messsignale selbst können in an sich bekannter Art und Weise mit Hilfe einer geeigneten Detektoreinrichtung, wie sie in verschiedenen Ausführungsformen als solche bekannt sind, erfasst werden. Hierbei besteht die Möglichkeit, die Auswerteeinrichtung zusammen mit der Detektoreinrichtung in der Analysevorrichtung zu integrieren. Aber auch eine Ausbildung der Auswerteeinrichtung getrennt von der Detektoreinrichtung kann vorgesehen sein, wobei die Auswerteeinrichtung die Messsignale in elektronischer Form von der Detektoreinrichtung direkt oder über eine Zwischenspeicherung der elektronischen Signale empfängt.

In einer bevorzugten Ausgestaltung werden Messsignale für ein Testsystem zur Ermittlung des Konzentrationsmaßes herangezogen, bei denen sich die Messbereiche quer zur Fließrichtung der Flüssigkeitsprobe auf dem Testsystem erstrecken. Es können Messsignale für Messbereiche verschiedener Formen ausgewertet werden, beispielsweise von Messbereichen, die eine Rechteck-, eine Kreis- oder eine Strichform aufweisen.

Besonders geeignet ist die Verwendung der vorgeschlagenen Technologien in Verbindung mit dem Bestimmen wenigstens einen Analyten in einer Körperflüssigkeitsprobe wie eine Blut- oder eine Urinprobe.

Das zur Konzentration des Analyten in der Flüssigkeitsprobe proportionale Konzentrationsmaß kann mit Hilfe der Auswerteeinrichtung wahlweise weiter verarbeitet werden, um eine tatsächliche Konzentration des Analyten als Absolut- oder Relativgröße zu ermitteln. In diesem Zusammenhang kann beispielsweise eine Weiterverarbeitung des Konzentrationsmaßes unter Verwendung von zuvor im Rahmen einer Kalibrierung oder Eichung erfassten Vergleichssignalen erfolgen. Die Unabhängigkeit des Konzentrationsmaßes von der Bindungseffizienz des Analyten in den Messbereichen oder Nachweiszonen führt zur Unabhängigkeit einer gegebenenfalls ermittelten tatsächlichen Konzentration von der Bindungseffizienz.

Eine bevorzugte Weiterbildung der Erfindung sieht vor, dass als das erste Messsignal ein erstes optisches Messsignal und als das zweite Messsignal ein zweites optisches Messsignal empfangen werden. Das erste und das zweite optische Messsignal können entsprechend einer Signalart ausgewählt aus der folgenden Gruppe von Signalarten gebildet sein: Fluoreszenzsignal, Transmissionssignal, Absorptionssignal und Reflexionssignal.

Bei einer zweckmäßigen Ausgestaltung der Erfindung kann vorgesehen sein, dass der erste und der zweite Messbereich benachbarte Bereiche auf dem Testsystem sind, so dass gilt $j = i+1$, und von der Auswerteeinrichtung das Konzentrationsmaß $N$ gemäß der folgenden vereinfachten Messsignalverknüpfung ermittelt wird:

$$N = \frac{(s_i)^{i+2}}{[s_i - s_{i+1}] \cdot (s_{i+1})^i} \; .$$

In Verbindung mit den vorteilhaften Ausgestaltungen der Analysevorrichtung zum Bestimmen eines Analyten in einer Flüssigkeitsprobe gelten die im Zusammenhang mit den vorangehend beschriebenen Ausführungen des Verfahrens gemachten Erläuterungen entsprechend. Bei der Analysevorrichtung kann darüber hinaus eine Detektoreinrichtung vorgesehen sein, die konfiguriert ist, das erste und das zweite Messsignal zu erfassen und an die Auswerteeinrichtung auszugeben. In einer bevorzugten Weiterbildung der Erfindung ist die Detektoreinrichtung eine optische Detektoreinrichtung, die konfiguriert ist, das erste und das zweite optische Messsignal einer Signalart ausgewählt aus der folgenden Gruppe von Signalarten entsprechend zu erfassen: Fluoreszenzsignal, Transmissionssignal, Absorptionssignal und Reflexionssignal.

In die Analysevorrichtung kann in einer vorteilhaften Ausführungsform der Erfindung ein Testsystem integriert sein, welches bevorzugt austauschbar oder für die Mehrfachnutzung konfiguriert ist. Unabhängig davon, ob das Testsystem in die Analysevorrichtung integriert ist oder hiervon getrennt gebildet ist, kann es sich bei dem Testsystem um ein Nachweissystem mit getrennt voneinander gebildeten Messbereichen oder einem zusammenhängenden Messbereich handeln, welcher messtechnisch so auswertbar ist, dass hintereinander angeordnete Messteilbereiche zum Erfassen der Messsignale auswertbar sind. Die hier vorgeschlagenen Technologien zur Ermittlung des Konzentrationsmaßes sind für beide Arten von Testsystemen nutzbar.

In einer Weiterbildung der Erfindung kann vorgesehen sein, dass das unter Verwendung des ersten und des zweiten Messsignals ermittelte Konzentrationsmaß zusammen mit dem Ergebnis weiterer derartiger Analysen, bei denen noch wenigstens ein Messsignal für einen weiteren Messbereich einbezogen wird, heranzuziehen, beispielsweise im Sinne einer Mittelwert-oder Durchschnittswertbildung. Auf diese Weise werden Messsignale für mehr als zwei Messbereiche des Testsystems verwendet.

Beschreibung bevorzugter Ausführungsbeispiele der Erfindung

Die Erfindung wird im Folgenden anhand von Ausführungsbeispielen unter Bezugnahme auf Figuren einer Zeichnung näher erläutert. Hierbei zeigen:

Fig. 1  eine schematische Darstellung eines Testsystems mit mehreren Messbereichen, die sich quer zu einer Ausbreitungsrichtung einer Flüssigkeitsprobe auf dem Testsystem erstrecken,

Fig. 2  eine grafische Darstellung für einen Signalabfall bei mehreren hintereinander liegen- den Messbereichen oder Nachweiszonen mit einer Strichform und

Fig. 3  eine grafische Darstellung für eine Fehlerfortpflanzung.

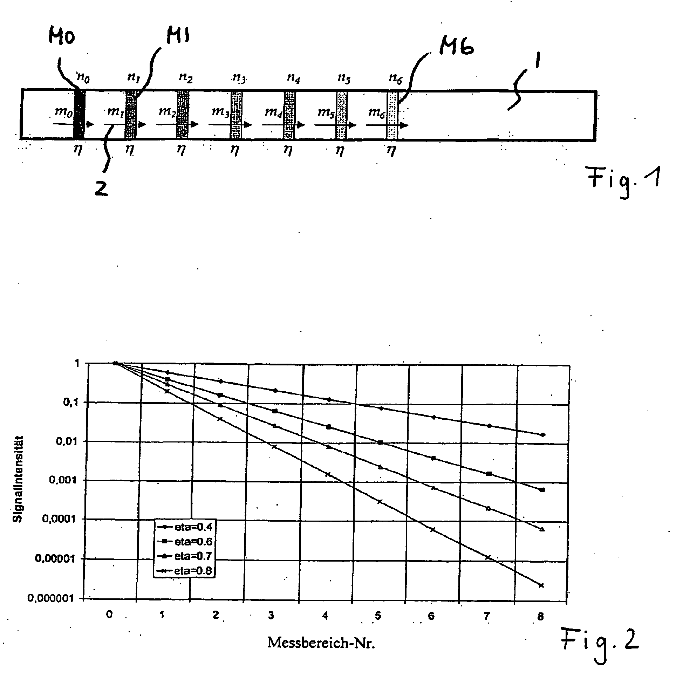

Fig. 1 zeigt eine schematische Darstellung eines Testsystems 1, auf dem entlang einer Ausbreitungsrichtung 2 und sich hierzu quer erstreckend mehrere Messbereiche oder Nachweiszonen M0, ..., M6 gebildet sind. Auf das Testsystem 1 kann eine Flüssigkeitsprobe, insbesondere eine Körperflüssigkeitsprobe, gegeben werden, die einen oder mehrere nachzuweisende Analyte enthält. Das eine oder die mehreren Analyte sind mit einem so genannten Label markiert. In den Messbereichen M0, ..., M6 sind dann analytspezifische Fängerstrukturen gebildet, an welche das Analyt bindet. Die Markierungen oder Label der gebundenen Analytkomplexe können dann messtechnisch für die Messbereiche M0, ..., M6 ausgewertet werden, bevorzugt mit Hilfe einer optischen Auswertung in Form von Absorptions-, Fluoreszenz-, Transmissions- oder Reflexionslicht. Hierzu werden Testlichtstrahlen auf die Messbereiche M0, ..., M6 eingestrahlt und zugehörige Messlichtstrahlen optisch detektiert mit Hilfe einer Detektoreinrichtung (nicht dargestellt).

Der unterschiedliche Schattierungsgrad der Messbereiche M0, ..., M6 in Fig. 1 zeigt den unterschiedlichen Umfang an, in welchem der Analyt in den Messbereichen M0, ..., M6 gebunden wird. Im Messbereich M0 erfolgt die Bindung im größten Umfang, wohingegen in dem zuletzt dargestellten Messbereich M6 nur noch eine geringe Menge des Analyten in der Flüssigkeitsprobe ist, weshalb dort nur wenige Analytmoleküle oder -strukturen gebunden werden.

Es sei nun $s_i$ ($i = 0, 1, ..., 6$) das für den Messbereich M0, ..., M6 erfasste Messsignal, also insbesondere ein optisches Messsignal, welches das Ausmaß oder den Umfang der Bindung von Analytstrukturen in dem Messbereich anzeigt. Hierbei ist es in einer Ausführungsform bevorzugt, Messsignale für Messbereiche heranzuziehen, in denen von einer Proportionalität zwischen der Anzahl der gebundenen, markierten Analytstrukturen und der Messsignalintensität ausgegangen werden kann. Letzteres kann beispielsweise insbesondere in den ersten Messbereichen nach dem Aufgabebereich des Testsystems 1 unter Umständen nicht der Fall sein, da dort aufgrund der hohen Zahl der gebundenen, markierten Analytstrukturen eine Messwertverfälschung auftreten kann. Die hohe Dichte von Labeln kann zu nichtlinearem optischen Verhalten führen.

Darüber hinaus werden zweckmäßigerweise bevorzugt Messsignale herangezogen, die im Dynamikbereich der Detektoreinrichtung liegen, das heißt dem Bereich, in welchem sich die messtechnisch relevante Antwortfunktion des Detektors linear verhält.

Es sei weiterhin η die (von $i$ unabhängige) Wahrscheinlichkeit (Bindungseffizienz), mit der ein über einen der Messbereiche wanderndes markiertes Analytelement in diesem gebunden wird, das heißt es gilt:

$$n_i = \eta \cdot m_i \qquad\qquad i = 0, 1, 2, \ldots, \qquad\qquad (1)$$

$m_i$ die Anzahl der über den $i$-ten Messbereich wandernden Label ist.

Das bevorzugt als optische Detektoreinrichtung ausgeführte Detektionssystem liefert dann bei der Vermessung der Messbereiche Messsignale $s_i$, die proportional zur Anzahl der in dem jeweilige Messbereich gefangenen Label ist, also der Anzahl der mit einem Label markierten und gebundenen Analytelemente. Es gilt:

$$s_i = C \cdot n_i \qquad\qquad i = 0, 1, 2, \ldots, \qquad\qquad (2)$$

wobei C ein Proportionalitätsfaktor ist.

Die auf diese Weise erfassten Messsignale $s_i$ für die Messbereiche werden dann mit Hilfe einer Auswerteeinrichtung (nicht dargestellt), bei der es sich beispielsweise um eine hardware- und / oder softwaretechnisch entsprechend konfigurierte Mikroprozessoreinheit handelt, gemäß der folgenden Signalverknüpfung verarbeitet:

$$N_i = \frac{(s_i)^{i+2}}{(s_i - s_{i+1}) \cdot (s_{i+1})^i} \qquad\qquad i = 0, 1, 2, \ldots$$

Das ermittelte Maß $N_i$ ist proportional zu $m_0$, also der Anzahl der Labelstrukturen, die den ersten Messbereich M0 erreichen, und nicht mehr von der Bindungseffizienz $\eta$ abhängig.

Dieses wird im Folgenden näher ausgeführt. Es gilt:

$$m_i = (m_0) \cdot (1 - \eta)^i \qquad\qquad i = 0, 1, 2, \ldots \qquad\qquad (4)$$

Damit ergibt sich $n_i = m_{i+1} - m_i = m_0 \cdot (1-\eta)^{i+1} - m_0 \cdot (1-\eta)^i$ zu:

$$n_i = m_0 \cdot \eta \cdot (1 - \eta)^i \qquad\qquad (5)$$

Gleichung (5) in Gleichung (3) eingesetzt ergibt:

$$N_i = \frac{\left(C \cdot m_0 \cdot \eta \cdot (1 - \eta)^i\right)^{i+2}}{\left(C \cdot m_0 \cdot \eta \cdot (1 - \eta)^i - C \cdot m_0 \cdot \eta \cdot (1 - \eta)^{i+1}\right) \cdot \left(C \cdot m_0 \cdot \eta \cdot (1 - \eta)^{i+1}\right)^i} \qquad\qquad (6)$$

Dies kann vereinfacht werden zu:

$$N_i = C \cdot m_0 \qquad\qquad i = 0, 1, 2, \ldots \qquad\qquad (7)$$

Die Messsignalverknüpfung gemäß Gleichung (3) bezieht sich auf die Auswertung benachbarter Messbereiche des Testsystems 1.

Eine zu einem gleichen Vorteil, nämlich der Unabhängigkeit von der Bindungseffizienz $\eta$ führende Messwertsignalauswertung lässt sich nun auch für nicht benachbarter Messbereiche des Testsystems 1 angeben, wobei ohne Beschränkung der Allgemeinheit der Proportionalitätsfaktor $C = 1$ gesetzt wurde:

Fall 1:    $j - i = 1$

$$\eta = 1 - \frac{n_j}{n_i} \qquad\qquad N_i = m_0 = \left[ \frac{s_i^{i+2}}{(s_j)^i \cdot (s_i - s_j)} \right]$$

Fall 2:    $j - i = 2$

$$\eta = 1 - \left(\frac{n_j}{n_i}\right)^{\frac{1}{2}} \qquad\qquad N_i = m_0 = \frac{s_i^{i+2}}{\left[ s_i - (s_i \cdot s_j)^{\frac{1}{2}} \right] \cdot (s_i \cdot s_j)^{\frac{i}{2}}}$$

(fortgesetzt)

Fall 3: $j - i = 3$

$$\eta = 1 - \left(\frac{n_j}{n_i}\right)^{\frac{1}{3}} \qquad N_i = m_0 = \frac{s_i^{i+2}}{\left[s_i - \left(s_i^{\,2} \cdot s_j\right)^{\frac{1}{3}}\right] \cdot \left(s_i^{\,2} \cdot s_j\right)^{\frac{i}{3}}}$$

Fall 4: $j - i = 4$

$$\eta = 1 - \left(\frac{n_j}{n_i}\right)^{\frac{1}{4}} \qquad N_i = m_0 = \frac{s_i^{i+2}}{\left[s_i - \left(s_i^{\,3} \cdot s_j\right)^{\frac{1}{4}}\right] \cdot \left(s_i^{\,3} \cdot s_j\right)^{\frac{i}{4}}}$$

Fall 5: $j - i = 5$

$$\eta = 1 - \left(\frac{n_j}{n_i}\right)^{\frac{1}{5}} \qquad N_i = m_0 = \frac{s_i^{i+2}}{\left[s_i - \left(s_i^{\,4} \cdot s_j\right)^{\frac{1}{5}}\right] \cdot \left(s_i^{\,4} \cdot s_j\right)^{\frac{i}{5}}}$$

Der Fall 1 bezieht sich zum Vergleich hierbei nochmals auf benachbarte Messbereiche, also $j = i+1$.

Von den vorangehenden Ausführungen ergibt sich nun in verallgemeinerter Form die folgende Messsignalverknüpfung:

$$N_{ij} = \frac{\left(s_j\right)^{i+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-i}}},$$

welche eine Messsignalverknüpfung für beliebige Messbereiche des Testsystems 1 darstellt, die die Ermittlung des hieraus abgeleiteten Konzentrationsmaßes $N_{ij}$ unabhängig von der Bindungseffizienz $\eta$ ermöglicht.

Der Vorteil der Verwendung von $N_i$ oder $N_{ij}$ als Maß für Analytkonzentration kann anhand der folgenden Betrachtungen weiter untersucht werden. Wird nach dem Fehlerfortpflanzungsgesetz die Auswirkung einer Variation der Bindungseffizienz $\eta$ betrachtet, so ergibt eine vergleichende Betrachtung für mit anderen Messsignalverknüpfungen:

| Variante | Verknüpfungsvorschrift | Fehlerfortpflanzung bei Variation von $\eta$ |
|---|---|---|
| Vorgeschlagene Messsignalverarbeitung | $N_i = \dfrac{s_i(\eta)^{i+2}}{\left(s_i(\eta) - s_{i+1}(\eta)\right) \cdot s_{i+1}(\eta)^{i}}$ | $\dfrac{dN_i}{N_i} = 0 \cdot \dfrac{d\eta}{\eta}$ |
| Auswertung nur eines Messbereiches - A1 | $N_i^{A1} = s_i(\eta)$ | $\dfrac{dN_i^{A1}}{N_i^{A1}} = \dfrac{1 - \eta(i+1)}{1 - \eta} \cdot \dfrac{d\eta}{\eta}$<br><br>Für $i=0$ ergibt sich:<br><br>$\dfrac{dN_0^{A1}}{N_0^{A1}} = 1 \cdot \dfrac{d\eta}{\eta}$ |

(fortgesetzt)

| Variante | Verknüpfungsvorschrift | Fehlerfortpflanzung bei Variation von $\eta$ |
|---|---|---|
| Additive Verknüpfung der Messsignale für benachbarte Messbereiche - A2 | $N_i^{A2} = s_i(\eta) + s_{i+1}(\eta)$ | $\dfrac{dN_i^{A2}}{N_i^{A2}} = \dfrac{\eta \cdot (\eta - 2) \cdot (i+2) + 2}{(\eta - 2) \cdot (\eta - 1)} \cdot \dfrac{d\eta}{\eta}$ <br><br> Für $i=0$ ergibt sich: <br><br> $\dfrac{dN_0^{A2}}{N_0^{A2}} = 2 \cdot \dfrac{1 - \eta}{2 - \eta} \dfrac{d\eta}{\eta}$ |
| Subtraktionsverknüpfung der Messsignale für benachbarte Messbereiche - A3 | $N_i^{A3} = s_i(\eta) - s_{i+1}(\eta)$ | $\dfrac{dN_i^{A3}}{N_i^{A3}} = \left( 2 - \dfrac{i \cdot \eta}{(1 - \eta)} \right) \cdot \dfrac{d\eta}{\eta}$ <br><br> Für $i=0$ ergibt sich: <br><br> $\dfrac{dN_0^{A3}}{N_0^{A3}} = 2 \cdot \dfrac{d\eta}{\eta}$ |

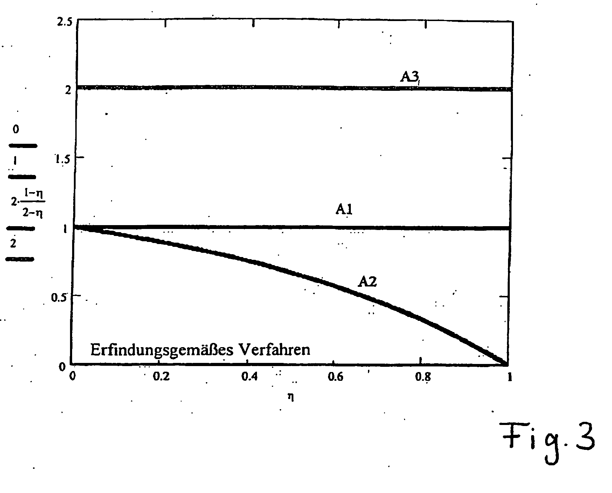

Fig. 3 ist eine grafische Darstellung für eine Fehlerfortpflanzung, welche die unterschiedlichen Fehlerfortpflanzung visualisiert für den Fall $i=0$.

Es ergibt sich weiterhin, dass die Messsignale bei der Vermessung der Nachweiszone um so geringer wird, je weiter hinten (in Fließ- / Ausbreitungsrichtung gesehen) die einzelne Nachweiszone liegt. Die bevorzugt optisch ausgeführte Detektion kann so eingestellt werden, dass bei sehr hohen Analytkonzentrationen die Anzahl der Label Messbereich $n_0$ so hoch ist, dass das Detektionssystem bei diesen Messbereichen in der Begrenzung ist, also eine Sättigung des Detektors auftritt. In diesem Fall würde $N_i$ aus den Messsignalen für zwei Nachweiszonen oder Messbereiche ermittelt, bei denen das Messsignal soweit abgefallen ist, dass es in den optimalen Arbeitsbereich des Detektors fällt, also zweckmäßig den linearen Arbeitsbereich. Umgekehrt würden sehr niedrige Konzentrationen aus den Messsignalen der letzten beiden Messbereiche ermittelt.

Die damit verbundene Dynamikerweiterung ist in Fig. 2 visualisiert. Es ist der Abfall der Anzahl der gebundenen Label (bzw. des Messsignals) gemäß Gleichung (5) mit steigender Ordnungszahl des Messbereiches für verschiedene Werte von $\eta$ gezeigt. Der Darstellung in Fig. 2 lässt sich entnehmen, dass sich mit fünf Messbereichen bei einer Bindungseffizienz von $\eta = 0.8$ die Dynamik der Detektion um 2.5 Größenordnungen steigern lässt.

**Patentansprüche**

**1.** Verfahren zum Bestimmen eines Analyten in einer Flüssigkeitsprobe, insbesondere einer Körperflüssigkeitsprobe, mit Hilfe einer Analysevorrichtung, bei dem:

- von einer Auswerteeinrichtung, die von der Analysevorrichtung umfasst ist, ein erstes Messsignal $s_i$ ($i = 0$, 1, ...) und ein zweites Messsignal $s_j$ ($j = 1, 2, ...; j > i$) für ein Testsystem mit mehreren nacheinander angeordneten und von einer Flüssigkeitsprobe mit einem Analyten nach einem Aufgeben auf das Testsystem durchlaufbaren Messbereichen empfangen werden, wobei das erste Messsignal $s_i$ einen in einem ersten Messbereich gebundenen Teil des Analyten und das zweite Messsignal $s_j$ einen in einem zweiten Messbereich, der dem ersten Messbereich auf dem Testsystem nachgelagert ist, gebundenen Teil des Analyten nach der Aufgabe der Flüssigkeitsprobe auf das Testsystem anzeigen, und
- von der Auswerteeinrichtung ein mit der Konzentration des Analyten in der Flüssigkeitsprobe korrelierendes Konzentrationsmaß $N$ ermittelt und nach einer wahlweisen Weiterverarbeitung ausgegeben wird,

**dadurch gekennzeichnet, dass** das Konzentrationsmaß $N$ gemäß der folgenden Messsignalverknüpfung ermittelt wird:

$$N = \frac{(s_j)^{j+2}}{\left[ s_i - \left( (s_i)^{j-i-1} \cdot s_j \right)^{\frac{1}{j-i}} \right] \cdot \left( (s_i)^{j-i-1} \cdot s_j \right)^{\frac{i}{j-1}}} \cdot$$

**2.** Verfahren nach Anspruch 1, **dadurch gekennzeichnet, dass** als das erste Messsignal ein erstes optisches Messsignal und als das zweite Messsignal ein zweites optisches Messsignal empfangen werden.

**3.** Verfahren nach Anspruch 1 oder 2, **dadurch gekennzeichnet, dass** der erste und der zweite Messbereich benachbarte Bereiche auf dem Testsystem sind, so dass gilt $j = i+1$, und von der Auswerteeinrichtung das Konzentrationsmaß $N$ gemäß der folgenden vereinfachten Messsignalverknüpfung ermittelt wird:

$$N = \frac{(s_i)^{i+2}}{[s_i - s_{i+1}] \cdot (s_{i+1})^i} \cdot$$

**4.** Analysevorrichtung zum Bestimmen eines Analyten in einer Flüssigkeitsprobe, insbesondere einer Körperflüssigkeitsprobe, mit einer Auswerteeinrichtung, die konfiguriert ist, ein erstes Messsignal $s_i$ ($i = 0, 1, ...$) und ein zweites Messsignal $s_j$ ($j = 1, 2, ...; j > i$) für ein Testsystem mit mehreren nacheinander angeordneten und von einer Flüssigkeitsprobe mit einem Analyten nach einem Aufgeben auf das Testsystem durchlaufbaren Messbereichen zu empfangen, wobei das erste Messsignal $s_i$ einen in einem ersten Messbereich gebundenen Teil des Analyten und das zweite Messsignal $s_j$ einen in einem zweiten Messbereich, der dem ersten Messbereich auf dem Testsystem nachgelagert ist, gebundenen Teil des Analyten nach der Aufgabe der Flüssigkeitsprobe auf das Testsystem anzeigen und wobei die Auswerteeinrichtung weiterhin konfiguriert ist, ein mit der Konzentration des Analyten in der Flüssigkeitsprobe korrelierendes Konzentrationsmaß $N$ zu ermitteln und auszugeben, **dadurch gekennzeichnet, dass** die Auswerteeinrichtung konfiguriert ist, das Konzentrationsmaß $N$ gemäß der folgenden Messsignalverknüpfung zu ermitteln:

$$N = \frac{(s_j)^{j+2}}{\left[ s_i - \left( (s_i)^{j-i-1} \cdot s_j \right)^{\frac{1}{j-i}} \right] \cdot \left( (s_i)^{j-i-1} \cdot s_j \right)^{\frac{i}{j-1}}} \cdot$$

**5.** Analysevorrichtung nach Anspruch 4, **dadurch gekennzeichnet, dass** die Auswerteeinrichtung weiterhin konfiguriert ist, das erste Messsignal ein erstes optisches Messsignal und als das zweite Messsignal ein zweites optisches Messsignal zu empfangen.

**6.** Analysevorrichtung nach Anspruch 4 oder 5, **dadurch gekennzeichnet, dass** die Auswerteeinrichtung weiterhin konfiguriert ist, das Konzentrationsmaß $N$ gemäß der folgenden vereinfachten Messsignalverknüpfung zu ermitteln:

$$N = \frac{(s_i)^{i+2}}{[s_i - s_{i+1}] \cdot (s_{i+1})^i} \, ,$$

wenn der erste und der zweite Messbereich benachbarte Bereiche auf dem Testsystem sind, so dass gilt $j = i+1$.

**7.** Analysevorrichtung nach mindestens einem der Ansprüche 4 bis 6, **gekennzeichnet durch** eine Detektoreinrichtung, die konfiguriert ist, das erste und das zweite Messsignal zu erfassen und an die Auswerteeinrichtung auszugeben.

8.  Analysevorrichtung nach Anspruch 7, **dadurch gekennzeichnet, dass** die Detektoreinrichtung eine optische Detektoreinrichtung ist, die konfiguriert ist, das erste und das zweite optische Messsignal einer Signalart ausgewählt aus der folgenden Gruppe von Signalarten entsprechend zu erfassten: Fluoreszenzsignal, Transmissionssignal, Absorptionssignal und Reflexionssignal.

9.  Analysevorrichtung nach mindestens einem der Ansprüche 4 bis 8, **gekennzeichnet** t durch ein integriertes Testsystem.

10. Computerprogramm-Produkt zum Bestimmen eines Analyten in einer Flüssigkeitsprobe, insbesondere einer Körperflüssigkeitsprobe, in einer Auswerteeinrichtung, wobei das Produkt die folgenden Mittel umfasst:

> - auf einem elektronischen Speichermittel aufgezeichnete Mittel zum Empfangen eines ersten Messsignals $s_i$ ($i = 0, 1, ...$) und eines zweiten Messsignals $s_j$ ($j = 1, 2, ...; j > i$) für ein Testsystem mit mehreren nacheinander angeordneten und von einer Flüssigkeitsprobe mit einem Analyten nach einem Aufgeben auf das Testsystem durchlaufbaren Messbereichen, wobei das erste Messsignal $s_i$ einen in einem ersten Messbereich gebundenen Teil des Analyten und das zweite Messsignal $s_j$ einen in einem zweiten Messbereich, der dem ersten Messbereich auf dem Testsystem nachgelagert ist, gebundenen Teil des Analyten nach der Aufgabe der Flüssigkeitsprobe auf das Testsystem anzeigen,
> - auf dem elektronischen Speichermittel aufgezeichnete Mittel zum Ermitteln eines mit der Konzentration des Analyten in der Flüssigkeitsprobe korrelierenden Konzentrationsmaßes $N$, und
> - auf dem elektronischen Speichermittel aufgezeichnete Mittel zum Ausgeben des Konzentrationsmaßes $N$ nach einer wahlweisen Weiterverarbeitung,

> **dadurch gekennzeichnet, dass** die auf dem elektronischen Speichermittel aufgezeichneten Mittel zum Ermitteln des Konzentrationsmaßes $N$ konfiguriert sind, das Konzentrationsmaß $N$ gemäß der folgenden Messsignalverknüpfung zu ermitteln:

$$N = \frac{\left(s_j\right)^{i+2}}{\left[ s_i - \left(\left(s_i\right)^{j-i-1} \cdot s_j\right)^{\frac{1}{j-i}} \right] \cdot \left(\left(s_i\right)^{j-i-1} \cdot s_j\right)^{\frac{i}{j-1}}} \; .$$

## Claims

1.  Method for the determination of an analyte in a liquid sample, especially a body liquid sample, with the aid of an analytical apparatus in which:

> - a first measuring signal $s_i$ ($i = 0, 1, ...$) and a second measuring signal $s_j$ ($j = 1, 2, ...; j > i$) for a test system with several successively arranged measuring areas through which a liquid sample with an analyte can flow after being applied on the test system are received by an evaluation apparatus comprised by the analytical apparatus, wherein the first measuring signal $s_i$ indicates a part of the analyte bound in a first measuring area and the second measuring signal $s_j$ indicates a part of the analyte bound in a second measuring area that is arranged downstream the first measuring area on the test system after the application of the liquid sample on the test system, and
> - a concentration measure $N$ correlating with the concentration of the analyte in the liquid sample is determined by the evaluation apparatus and is outputted after a selective further processing,

> **characterized in that** the concentration measure $N$ is determined in accordance with the following linkage of measuring signals:

$$N = \frac{\left(s_j\right)^{i+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-1}}} .$$

2. Method according to Claim 1, **characterized in that** a first optical measuring signal is received as the first measuring signal and a second optical measuring signal is received as the second measuring signal.

3. Method according to Claim 1 or 2, **characterized in that** the first and the second measuring areas are adjacent areas on the test system, so that $j = i+1$, and the concentration measure $N$ is determined by the evaluation apparatus in accordance with the following simplified linking of measuring signals:

$$N = \frac{\left(s_i\right)^{i+2}}{\left[s_i - s_{i+1}\right] \cdot \left(s_{i+1}\right)^{i}} .$$

4. Analytical apparatus for the determination of an analyte in a liquid sample, especially a body liquid sample, with an evaluation apparatus that is configured to receive a first measuring signal $s_i$ ($i = 0, 1, ...$) and a second measuring signal $s_j$ ($j = 1, 2, ...; j > i$) for a test system with several successively arranged measuring areas through which a liquid sample with an analyte can flow after being applied on the test system, wherein the first measuring signal $s_i$ indicates a part of the analyte bound in a first measuring area and the second measuring signal $s_j$ indicates a part of the analyte bound in a second measuring area that is arranged downstream the first measuring area on the test system after the application of the liquid sample on the test system, and wherein the evaluation apparatus is furthermore configured for determining and outputting a concentration measure $N$ correlating with the concentration of the analyte in the liquid sample **characterized in that** the evaluation apparatus is configured to determine the concentration measure $N$ in accordance with the following linkage of measuring signals:

$$N = \frac{\left(s_j\right)^{i+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-1}}} .$$

5. Analytical apparatus according to Claim 4, **characterized in that** the evaluation apparatus is furthermore configured to receive a first optical measuring signal as the first measuring signal and a second optical measuring signal as the second measuring signal.

6. Analytical apparatus according to Claim 4 or 5, **characterized in that** the evaluation apparatus is furthermore configured to determine the concentration measure $N$ in accordance with the following simplified linking of measuring signals:

$$N = \frac{\left(s_i\right)^{i+2}}{\left[s_i - s_{i+1}\right] \cdot \left(s_{i+1}\right)^{i}} ,$$

when the first and the second measuring areas are adjacent areas on the test system, so that $j = i+1$.

7. Analytical apparatus according to at least one of the Claims 4 to 6, **characterized by** a detector apparatus which is configured to detect the first and the second measuring signal and to output them to the evaluation apparatus.

8. Analytical apparatus according to Claim 7, **characterized in that** the detector apparatus is an optical detector apparatus which is configured to appropriately detect the first and the second optical measuring signal of a signal type selected from the following group of signal types: fluorescence signal, transmission signal, absorption signal and reflection signal.

9. Analytical apparatus according to at least one of the Claims 4 to 8, **characterized by** an integrated test system.

10. Computer program product for the determination of an analyte in a liquid sample, especially a body liquid sample, in an evaluation apparatus, wherein the product comprises the following means:

- means recorded on an electronic storage means for receiving a first measuring signal $s_i$ ($i$ = 0, 1, ...) and a second measuring signal $s_j$ ($j$ = 1, 2, ...; $j > i$) for a test system with several successively arranged measuring areas through which a liquid sample with an analyte can flow after being applied on the test system, wherein the first measuring signal $s_i$ indicates a part of the analyte bound in a first measuring area and the second measuring signal $s_j$ indicates a part of the analyte bound in a second measuring area that is arranged downstream the first measuring area on the test system after the application of the liquid sample on the test system,
- means recorded on the electronic storage means for determining a concentration measure $N$ correlating with the concentration of the analyte in the liquid sample, and
- means recorded on the electronic storage means for outputting the concentration measure $N$ after a selective further processing,

**characterized in that** the means recorded on the electronic storage means for determining the concentration measure $N$ are configured to determine the concentration measure $N$ in accordance with the following linking of measuring signals:

$$N = \frac{\left(s_j\right)^{j+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-1}}} \ .$$

**Revendications**

1. Procédé de détermination d'un analyte dans un échantillon de liquide, en particulier un échantillon de liquide de l'organisme, à l'aide d'un dispositif d'analyse, dans lequel

- un équipement d'évaluation que comprend le dispositif d'analyse reçoit un premier signal de mesure $s_i$ ($i$ = 0, 1, ...) et un deuxième signal de mesure $s_j$ ($j$ = 1, 2, ...; $j > i$) pour un système de test avec plusieurs plages de mesure disposés l'un après l'autre et pouvant être traversés par un échantillon de liquide avec un analyte après alimentation dans le système de test, étant donné que le premier signal de mesure $s_i$ indique une partie de l'analyte lié dans une première plage de mesure et le deuxième signal de mesure $s_j$ une partie liée de l'analyte dans une deuxième plage de mesure en aval de la première plage de mesure dans le système de test après alimentation de l'échantillon de liquide dans le système de test, et
- l'équipement d'évaluation détermine une valeur de concentration $N$ en corrélation avec la concentration de l'analyte dans l'échantillon de liquide et la sort après un traitement ultérieur facultatif,

**caractérisé en ce que** la valeur de concentration $N$ est déterminée conformément à l'équation de signal de mesure suivante :

$$N = \frac{\left(s_j\right)^{j+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-i}}} .$$

**2.** Procédé selon la revendication 1, **caractérisé en ce que** sont reçus comme premier signal de mesure un premier signal de mesure optique et comme deuxième signal de mesure un deuxième signal de mesure optique.

**3.** Procédé selon la revendication 1 ou 2, **caractérisé en ce que** la première et la deuxième plage de mesure sont des plages voisines dans le système de test, de telle sorte que $j = i + 1$, et l'équipement d'évaluation détermine la valeur de concentration $N$ conformément à l'équation de signal de mesure simplifiée suivante :

$$N = \frac{\left(s_i\right)^{j+2}}{\left[s_i - s_{i+1}\right] \cdot \left(s_{i+1}\right)^{j}} .$$

**4.** dispositif d'analyse pour la détermination d'un analyte dans un échantillon de liquide, en particulier un échantillon de liquide de l'organisme, avec un équipement d'évaluation configuré pour recevoir un premier signal de mesure $s_i$ ($i = 0, 1, ...$) et un deuxième signal de mesure $s_j$ ($j = 1, 2, ...; j > i$) pour un système de test avec plusieurs plages de mesure disposés l'un après l'autre et pouvant être traversés par un échantillon de liquide avec un analyte après alimentation dans le système de test, étant donné que le premier signal de mesure $s_i$ indique une partie de l'analyte lié dans une première plage de mesure et le deuxième signal de mesure $s_j$ une partie liée de l'analyte dans une deuxième plage de mesure en aval de la première plage de mesure dans le système de test après alimentation de l'échantillon de liquide dans le système de test, et étant donné que l'équipement d'évaluation est en outre configuré pour déterminer et sortir une valeur de concentration $N$ en corrélation avec la concentration de l'analyte dans l'échantillon de liquide, **caractérisé en ce que** l'équipement d'évaluation est configuré pour déterminer la valeur de concentration $N$ conformément à l'équation de signal de mesure suivante :

$$N = \frac{\left(s_j\right)^{j+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-i}}} .$$

**5.** Dispositif d'analyse selon la revendication 4, **caractérisé en ce que** l'équipement d'évaluation est en outre configuré pour recevoir comme premier signal de mesure un premier signal de mesure optique et comme deuxième signal de mesure un deuxième signal de mesure optique.

**6.** Dispositif d'analyse selon la revendication 4 ou 5, **caractérisé en ce que** l'équipement d'évaluation est en outre configuré pour déterminer la valeur de concentration $N$ conformément à l'équation de signal de mesure simplifiée suivante :

$$N = \frac{\left(s_i\right)^{j+2}}{\left[s_i - s_{i+1}\right] \cdot \left(s_{i+1}\right)^{j}} ,$$

où la première et la deuxième plage de mesure sont des plages voisines dans le système de test, avec $j = i + l$.

7. Dispositif d'analyse selon au moins l'une quelconque des revendications 4 à 6, **caractérisé par** un équipement détecteur qui est configuré pour saisir les premier et deuxième signaux de mesure et les transmettre à l'équipement d'évaluation.

8. Dispositif d'analyse selon la revendication 7, **caractérisé en ce que** l'équipement détecteur est un équipement détecteur optique configuré pour saisir les premier et deuxième signaux de mesure optiques d'un type de signal sélectionné dans le groupe de types de signaux suivant : signal de fluorescence, signal de transmission, signal d'absorption et signal de réflexion.

9. Dispositif d'analyse selon au moins l'une quelconque des revendications 4 à 8, **caractérisé par** un système de test intégré.

10. Programme d'ordinateur pour la détermination d'un analyte dans un échantillon de liquide, en particulier un échantillon de liquide de l'organisme, dans un équipement d'évaluation, le produit comprenant les moyens suivants :

- moyens enregistrés sur un moyen d'enregistrement électronique pour la réception d'un premier signal de mesure $s_i (i = 0, 1, ...)$ et d'un deuxième signal de mesure $s_j$ ($j = 1, 2, ...$ ; $j > i$) pour un système de test avec plusieurs plages de mesure disposés l'un après l'autre et pouvant être traversés par un échantillon de liquide avec un analyte après alimentation dans le système de test, étant donné que le premier signal de mesure $s_i$ indique une partie de l'analyte lié dans une première plage de mesure et le deuxième signal de mesure $s_j$ une partie liée de l'analyte dans une deuxième plage de mesure en aval de la première plage de mesure dans le système de test après alimentation de l'échantillon de liquide dans le système de test,
- moyens enregistrés sur le moyen d'enregistrement électronique pour la détermination d'une valeur de concentration $N$ en corrélation avec la concentration de l'analyte dans l'échantillon de liquide, et
- moyens enregistrés sur le moyen d'enregistrement électronique pour la sortie de la valeur de concentration $N$ après un traitement ultérieur facultatif,

**caractérisé en ce que** les moyens enregistrés sur le moyen d'enregistrement électronique pour la détermination de la valeur de concentration $N$ sont configurés pour déterminer la valeur de concentration $N$ conformément à l'équation de signal de mesure suivante :

$$N = \frac{\left(s_i\right)^{j+2}}{\left[s_i - \left(\left(s_i\right)^{j-i-1} \cdot s_j\right)^{\frac{1}{j-i}}\right] \cdot \left(\left(s_i\right)^{j-i-1} \cdot s_j\right)^{\frac{i}{j-i}}}$$

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0690306 A1

**In der Beschreibung aufgeführte Nicht-Patentliteratur**

- **J. J. Holbrook.** Protein Fluorescence of Lactate Dehydrogenase. *Biochem. J.,* 1972, vol. 128, 921